## Europäisches Patentamt
## European Patent Office
## Office européen des brevets

(11) Publication number: **0 023 781**
**B1**

(12) **EUROPEAN PATENT SPECIFICATION**

(45) Date of publication of patent specification: **14.03.84**

(51) Int. Cl.³: **A 61 F 5/44**

(21) Application number: **80302370.4**

(22) Date of filing: **11.07.80**

(54) Detachable filter and ostomy bag including same.

(30) Priority: **16.07.79 GB 7924699**
**23.06.80 GB 8020531**

(43) Date of publication of application:
**11.02.81 Bulletin 81/6**

(45) Publication of the grant of the patent:
**14.03.84 Bulletin 84/11**

(84) Designated Contracting States:
**AT BE CH DE FR IT LI LU NL SE**

(56) References cited:
**GB - A - 1 117 204**
**GB - A - 1 301 101**
**GB - A - 1 379 464**
**GB - A - 1 405 032**
**US - A - 2 054 535**
**US - A - 2 555 086**

(73) Proprietor: **KINGSDOWN MEDICAL CONSULTANTS LIMITED**
**Blackfriars House 19, New Bridge Street**
**London, EC4Y 6BY (GB)**

(72) Inventor: **Steer, Peter Leslie**
**Malt House Works Station Road**
**East Grinstead, Sussex (GB)**
Inventor: **Edwards, John Victor**
**5, Lodge Close**
**East Grinstead, Sussex (GB)**

(74) Representative: **Cook, Anthony John et al,**
**D. YOUNG & CO. 10, Staple Inn**
**London, WC1V 7RD (GB)**

Courier Press, Leamington Spa, England.

## Detachable Filter and Ostomy Bag Including Same

This invention relates to a detachable filter for an ostomy bag and to an ostomy bag including such a filter. In this Specification, the term "ostomy bag" is used to mean a colostomy or ileostomy or other kind of bag intended to be worn by a user to receive waste material expelled from the stoma. It is well known that flatus gives rise to problems and numerous attempts have been made to allow wind to escape from the bag and to remove indesirable odours from the gas. For example British Patent Specification No. 1,117,204 proposes providing a permanent opening in a wall of the bag facing away from the wearer and covering the opening with a filter. British Patent Specification No. 1,374,464 suggests that a vent aperture should be located in the bag at a region which is overlaid by a flange used to secure the bag to a patient.

One advantage of the attachable and detachable filter disclosed herein is that manufacture of ostomy bags is very substantially simplified. Also, problems arising from application of activated carbon powder to a patch secured to the bag by adhesive, such as is disclosed in Patent 1,379,464 are avoided. Also, while this Patent discloses releasably sealing a vent aperture by means of a coating of adhesive material and by applying a flange thereto, but it will be understood that the effectiveness of the sealing over a period of time may decrease as the adhesive layer becomes dirty or otherwise loses its stickiness.

According to the present invention, there is provided an attachable and detachable filter for use with an ostomy bag, the filter including a plastics housing member having two portions, characterised that the two portions are hinged together by an integral hinge, each portion having a peripheral wall, at least one of the walls having at least one aperture therein, one portion carrying a sharp-pointed and resilient-headed stud which is a push fit in relation to a hole or recess in the other to clamp together the two portions, there being at least one layer of gas-filtering material contained within one or other of both the housing portions.

The aperture may have an inwardly-extending rib which is deformed by the stud as the stud is pushed in the aperture, and consequently grips the stud.

The gas filtering material may be carbon cloth as defined in British Patent Specification No. 1,301,101. Alternatively it may be a woven or non-woven fibrous material impregnated with activated carbon particles.

In use, such a filter is applied to a bag by folding it over a top edge of the bag, forcing the pointed head of the stud through the two bag walls and then through the aperture in the other portion. This makes a hole in both the back and front bag walls of the upper region of the bag and any gas in the bag can pass out through one or other of these holes. The gas then arrives at the space around the stud within the two housing portions, and its only path to the exterior is past the carbon cloth or the carbon-impregnated material and through the aperture or apertures in the peripheral wall. The activated carbon of the cloth or the carbon particles serve to deodorise the issuing gas.

The invention will be better understood from the following description of an illustrative and non-limiting example thereof, given with reference to the accompanying drawings in which:—

Figure 1 is a central vertical section through one example of attachable and detachable filter shown in the open position;

Figure 2 is a plan view of the filter of Figure 1;

Figure 3 is an end elevation looking in the direction III in Figure 2;

Figure 4 is a detail on an enlarged scale showing one possible construction of the press stud, and

Figure 5 is a detail on an enlarged scale showing one possible construction of the integral hinge; and

Figure 6 is a cross sectional view similar to Figure 1 but showing the second example of the invention.

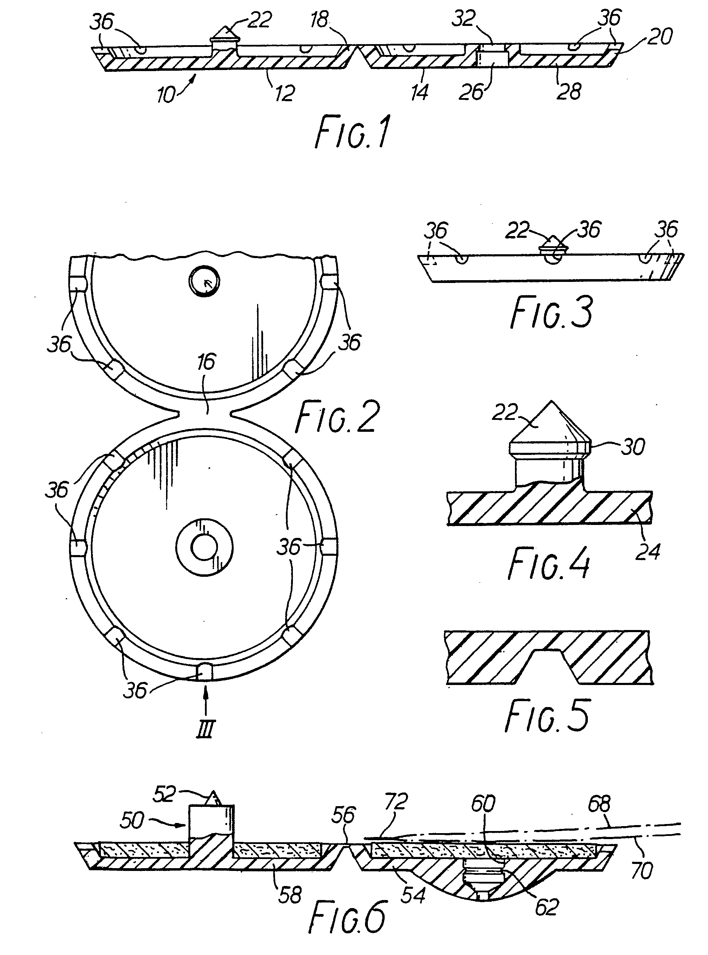

The illustrated attachable and detachable filter includes a plastics housing member 10 having two portions 12 and 14 hinged together by an integral hinge 16, Figure 2, and each portion has a peripheral wall 18, 20. The portion 12 carries a sharp pointed resilient headed stud 22 which extends upwardly from the centre of the flat wall 24 of the first portion. A corresponding recess 26 is provided at the centre of a flat wall 28 of the second portion 14. The head 22 of the stud is enlarged as shown at 30 and the stud is made of resilient material so that it can be forced through a hole 32 in the base wall of the recess 26 and so hold the two portions 12 and 14 together in the closed condition of the attachable and detachable filter.

As shown, each of the portions 12 and 14 of the filter housing have radial apertures 36 therein, but it may be sufficient if these are provided in the peripheral wall of only one of the two portions. In use these apertures function as gas exit apertures, and is may be sufficient to provide one such aperture.

In either or both of the filter housing portions 12 and 14 is disposed a layer of gas filtering material. This may for example be a woven or non-woven fibrous material impregnated with activated carbon particles, or it may be a carbon cloth as described and claimed in British Patent Specification No. 1,301,101.

One suitable material for the housing 10 is a polypropylene copolymer, but the invention is not limited to the use of this material and it will be appreciated that other plastics materials are suitable. Also although an integral hinge 16 has been described, it will be appreciated that alternatively a separate hinge could be fixed to each housing portion 12 and 14.

In use, the attachable and detachable filter housing is clipped over the top edge of an ostomy bag, with the pointed head 22 being forced through both the bag walls so making an orifice in each of them. The head 22 is forced into the recess 26, so holding the filter housing in a doubled over condition firmly clipped onto the ostomy bag. In this way, an orifice in each bag wall has been punctured and allows any gases in the ostomy bag to escape into the space defined between the closed housing portions 12 and 14. It can then pass to the exterior by travelling outwardly radially over or past the carbon cloth material (or past the non-woven or woven material carrying activated carbon particles) and thence to the apertures 36. In this way, it is ensured that the escaping gases are necessarily forced into contact with activated carbon over a considerable distance along their exit path. In this way the escaping gases are deodorised.

The attachable and detachable filter illustrated in Figure 6 is similar in essential features to that shown in Figures 1—5. However, the filter shown in Figure 6 has a stud 50 which has a main body part 52 which is cylindrical, the latter carrying a pointed tip. One portion 54 of the housing is integrally hinged by hinge 56 to the other portion 58, the latter being integral with the stud 50. A recess 60 provided in the portion 54 has an inwardly extending peripheral rib 62 which is deformable by the stud 50 when the two portions are closed together. This exerts a gripping action on the main body part 52 of the stud.

The rib 62 is moulded during the manufacture of the housing; injection moulding may be used for this purpose. Instead of a peripheral rib, one may employ a number of bumps or projections extending inwardly an appropriate distance from the internal surface of the cylindrical wall of the recess 60. The housing contains carbon cloth, or other filter material, in the same way as the housing shown in Figures 1—5. The housing of Figure 6 may be moulded from a polypropylene copolymer.

Two walls 68, 70 and the top edge 72 of an ostomy bag are indicated diagrammatically in the right hand side of Figure 6.

The filter may be readily detached and, if desired, fitted to a new bag.

While there have been disclosed (in Figures 1—5) a single sharp headed press stud and a single recess, it will be appreciated that more than one such stud and corresponding recess may be provided if desired. Naturally, while the first and second housing portions 12 and 14 have been shown as generally circular, they may be constructed of other shapes.

A slot, or more than one slot, may be provided in the shank of the stud 22 to allow gas passage in the event that the stud punches a hole in the bag wall whose edges closely embrace the stud shank.

## Claims

1. An attachable and detachable filter for use with an ostomy bag, the filter including a plastics housing member having two portions, characterised in that the two portions are hinged together by a hinge, each portion (10, 12; 54, 58) having a peripheral wall, at least one of the walls having at least one gas escape aperture (36) therein, one portion carrying a sharp-pointed stud (22; 50) which is a push fit in relation to a hole or recess (26, 32, 60) in the other to clamp together the two portions, there being at least one layer of gas-filtering material contained within one or other or both the housing portions.

2. A filter according to claim 1 in which the stud has a longitudinal slot in a shank portion thereof.

3. A filter according to claim 1 or 2 in which there are a plurality of apertures in one of the housing portions and no apertures in the other.

4. A filter according to claim 3 in which the apertures (36) are constituted by recesses in that edge of the wall further from the junction of the wall with remainder of the plastics housing member portion.

5. A filter according to any preceding claim in which the housing member (10; 54, 58) is made of polypropylene copolymer.

6. A filter according to claim 1 or 2 in which the stud is generally cylindrical and the hole (26, 32) has an inwardly extending rib.

7. A filter according to claim 1 or 2 in which the stud (50) is generally cylindrical and the recess (60) has a wall with a projection (62) extending inwardly to grip the stud when the housing is closed.

8. A filter according to any one of the preceding claims in which the hinge is integral with the portions of the housing.

9. An ostomy bag in combination with a filter according to any one of claims 1—7.

## Revendications

1. Filtre attachable et détachable destiné à être utilisé avec un sac d'ostomie, ce filtre comportant un logement en matière plastique en deux parties, caractérisé en ce que les deux parties sont articulées l'une sur l'autre par un charnière, chaque partie (10, 12; 54, 58) ayant une paroi périphérique, au moins une ouverture d'échappement de gaz (36) étant pratiquée dans au moins une des parois, une partie portant un bouton ou tenon (22; 50) à extrémité pointue qui s'adapte par pression dans un trou ou évidement (26, 32, 60) ménagé dans l'autre partie de façon que les deux parties soient maintenues emboîtées l'une dans l'autre, au moins une couche de matière de filtration de gaz, contenue dans l'une ou l'autre des parties du logement, ou dans les deux, étant prévue.

2. Filtre selon la revendication 1, dans lequel le bouton ou tenon comporte une fente longitudinale dans sa partie cylindrique.

3. Filtre selon la revendication 1 ou 2, dans lequel il y a plusieurs ouvertures dans l'une des parties du logement et aucune ouverture dans l'autre.

4. Filtre selon la revendication 3, dans lequel les ouvertures (36) sont constituées par des évidements ménagés dans le bord de la paroi le plus éloigné dela jonction de la paroi avec le reste de la partie correspondante du logement en matière plastique.

5. Filtre selon l'une quelconque des revendications précédentes, dans lequel le logement (10; 54, 58) est fait d'un copolymère de polypropylène.

6. Filtre selon la revendication 1 ou 2, dans lequel le bouton ou tenon a une forme générale cylindrique et le trou (26, 32) comporte une nervure dirigée vers l'intérieur.

7. Filtre selon la revendication 1 ou 2, dans lequel le bouton ou tenon (50) a une forme générale cylindrique et l'évidement (60) comporte une paroi avec une protubérance (62) qui est dirigée vers l'intérieur de façon à enserrer le bouton ou tenon lorsque le logement est fermé.

8. Filtre selon l'une quelconque des revendications précédentes, dans lequel la charnière fait corps avec les deux parties du logement.

9. Sac d'ostomie combiné à un filtre selon l'une quelconque des revendications 1 à 7.

**Patentansprüche**

1. Aufsteckbarer und abnehmbarer Filter für Ostomiebeutel, wobei der Filter ein Kunststoffgehäuse aus zwei Teilen aufweist, dadurch gekennzeichnet, daß die beiden Teile mittels eines Gelenks aneinander angelenkt sind, daß jedes Teil (10, 12; 54, 58) am Umfang eine Wandung aufweist, daß mindestens eine der Wandungen mindestens eine Gasaustrittsöffnung (36) aufweist, daß ein Teil einen scharf zugespitzten Zapfen (22; 50) aufweist, der gegenüber einer Bohrung oder Aussparung (26, 32; 60) im anderen Teil eine Druckpassung bildet, um die beiden Teile miteinander zu verklemmen, und daß mindestens eine Schicht eines Gasfiltermaterials in mindestens einem der beiden Gehäuseteile enthalten ist.

2. Filter nach Anspruch 1, dadurch gekennzeichnet, daß der Zapfen im Schaft eine Längsrille aufweist.

3. Filter nach Anspruch 1 oder 2, dadurch gekennzeichnet, daß ein Gehäuseteil mehrere Öffnungen und das andere Gehäuseteil keine Öffnungen aufweist.

4. Filter nach Anspruch 3, dadurch gekennzeichnet, daß die Öffnungen (36) durch Aussparungen in von der Verbindung der Wandung mit dem übrigen Teil des Kunststoffgehäuses entfernten Abschnitt der Wandung gebildet werden.

5. Filter nach einem der vorstehenden Ansprüche, dadurch gekennzeichnet, daß das Gehäuse (10; 54, 58) aus einem Polypropylen-Copolymerisat besteht.

6. Filter nach Anspruch 1 oder 2, dadurch gekennzeichnet, daß der Zapfen im wesentlichen Zylindrisch ist und die Bohrung (26, 32) eine sich nach innen erstreckende Rippe aufweist.

7. Filter nach Anspruch 1 oder 2, dadurch gekennzeichnet, daß der Zapfen (50) im wesentlichen zylindrisch ist und daß die Bohrung (60) eine Wand mit einem nach innen sich erstreckenden Vorsprung (62) zum Festhalten des Zapfens bei geschlossenem Gehäuse aufweist.

8. Filter nach einen der vorstehenden Ansprüche, dadurch gekennzeichnet, daß das Gelenk mit den Gehäuseteilen einstückig ist.

9. Ostomiebeutel mit einem Filter nach einem der Ansprüche 1 bis 7.

FIG.1

FIG.2

FIG.3

FIG.4

FIG.5

FIG.6